# EUROPÄISCHE PATENTSCHRIFT

(11) **EP 2 595 943 B1**
(45) Veröffentlichungstag und Bekanntmachung des Hinweises auf die Patenterteilung: **15.04.2015**
(21) Anmeldenummer: 11735641.0
(22) Anmeldetag: 20.07.2011
(51) Int. Cl.: C07C 2/76

(54) **VERFAHREN ZUR HERSTELLUNG VON AROMATEN AUS METHAN**
METHOD FOR PRODUCING AROMATIC COMPOUNDS FROM METHANE
PROCÉDÉ POUR PRODUIRE DES AROMATES À PARTIR DE MÉTHANE

(30) Priorität: 21.07.2010 EP 10170257
(43) Veröffentlichungstag der Anmeldung: 29.05.2013
(73) Patentinhaber: BASF SE, 67056 Ludwigshafen (DE)
(72) Erfinder: SCHNEIDER, Christian, 68165 Mannheim (DE); AHRENS, Sebastian, 69168 Wiesloch (DE); BACHMANN, Kati, 68309 Mannheim (DE); COELHO TSOU, Joana, B-1190 Brüssel (BE); HEIDEMANN, Thomas, 68519 Viernheim (DE); WENTINK, Annebart Engbert, 68161 Mannheim (DE)
(86) Internationale Anmeldenummer: PCT/EP2011/062415
(87) Internationale Veröffentlichungsnummer: WO 2012/022569

(56) Entgegenhaltungen:
- US-A- 5 030 338

## Beschreibung

Die vorliegende Erfindung betrifft ein Verfahren zur Durchführung von endothermen, heterogen katalysierten Reaktionen, bei denen die Umsetzung der Edukte in Gegenwart einer Mischung aus inerten Wärmeüberträgerpartikeln und Katalysatorpartikeln erfolgt, wobei die Katalysatorpartikel in regelmäßigen Abständen in nicht-oxidativer Atmosphäre regeneriert werden und die benötigte Reaktionswärme durch Abtrennen der inerten Wärmeüberträgerpartikel, Aufheizen der Wärmeüberträgerpartikel in einer Aufheizzone und Rückführen der aufgeheizten Wärmeüberträgerpartikel in die Reaktionszone eingebracht wird. Besonders gut eignet sich das erfindungsgemäße Verfahren für die nicht-oxidative Dehydroaromatisierung von C₁-C₄-Aliphaten in Gegenwart von zeolithhaltigen Katalysatoren.

Bei vielen endothermen Reaktionen stellt die Zuführung der dafür benötigten Energie eine besondere Herausforderung dar. Wird die Reaktion indirekt beheizt, sind große Wärmeübertragungsflächen erforderlich, die das Verfahren apparativ und wirtschaftlich aufwändig gestalten. Darüber hinaus finden an den Wärmeübertragungsflächen aufgrund der höheren Temperaturen häufig unerwünschte Nebenreaktionen statt, beispielsweise Verkokung bei der Umsetzung von Kohlenwasserstoffen. Dies gilt unter anderem auch für die nicht-oxidative Dehydroaromatisierung von Methan (DHAM), die eine endotherme Reaktion und auf externe Wärmezufuhr angewiesen ist.

Eine Möglichkeit zum direkten Einbringen von Reaktionswärme ist die Verwendung von nicht an der Reaktion teilnehmenden Partikeln als Wärmeüberträgerpartikel, die in einem externen Reaktor ggf. gemeinsam mit Katalysatorpartikeln durch direkten Kontakt mit Verbrennungsabgasen bzw. durch direkte Verbrennung eines Brennstoffs auf eine Temperatur oberhalb der Reaktionstemperatur aufgeheizt werden und anschließend in die Reaktionszone zurückgeführt werden. Die für die Reaktion benötigte Energie wird anschließend durch direkten Kontakt der inerten Wärmeüberträgerpartikel mit den Katalysatorpartikeln übertragen. Derartige Verfahren, bei denen inerte Partikel zum Einkoppeln der Reaktionswärme eingesetzt werden, sind aus dem Stand der Technik bekannt.

Ein weiteres Problem bei vielen feststoffkatalysierten Reaktionen ist die zunehmende Deaktivierung des eingesetzten Katalysators, der regelmäßig regeneriert werden muss. So erfolgt bei der technischen Anwendung der Dehydroaromatisierung von C₁-C₄-Aliphaten unter nicht-oxidativen Bedingungen eine Verkokung der Katalysatoren, die in relativ kurzer Zeit die Aktivität des Katalysators herabsetzt, was zu kurzen Produktionszyklen und einem hohen Regenerierungsbedarf führt. Häufig geht mit der Verkokung eine verkürzte Lebensdauer des Katalysators einher. Die Regenerierung der Katalysatoren ist nicht unproblematisch, da für ein wirtschaftliches Verfahren zum einen regelmäßig die Ausgangsaktivitäten wieder herstellbar sein müssen und zum anderen dies über eine große Anzahl von Zyklen möglich sein muss.

Die Koksablagerungen wirken sich zudem ungünstig auf die Stoffbilanz bzw. die Ausbeute aus, da jedes Molekül Edukt, das in Koks umgewandelt wird, nicht mehr für die gewünschte Reaktion zu Aromaten zur Verfügung steht. Die bisher im Stand der Technik erreichten Koksselektivitäten liegen in den meisten Fällen bei über 20 % bezogen auf den umgesetzten Aliphaten.

Verfahren, bei denen die Reaktionswärme durch Aufheizen von Wärmeüberträgerpartikeln zugeführt wird und die Katalysatorpartikel regelmäßig regeneriert werden, sind bekannt.

In US 5,030,338 wird ein Verfahren zur aliphatischen Aromatisierung in Gegenwart von Zeolithen als Katalysatoren und inerten Teilchen beschrieben, bei dem die Mischung aus deaktiviertem Katalysator und inerten Partikeln aus der Reaktionszone abgezogen wird, die Mischung von anhaftenden Kohlenwasserstoffen durch Strippen befreit wird und die gestrippte Mischung in einen Strom, der vorwiegend Katalysatorteilchen enthält, und einen zweiten Strom, der im Wesentlichen Inertpartikel enthält, aufgetrennt wird. Der vorwiegend Katalysator enthaltende Strom wird in eine Regenerationszone überführt und mit einem sauerstoffhaltigen Gas regeneriert. Der zweite, vorwiegend die Inertpartikel enthaltende Strom wird in eine Verbrennungszone eingebracht, in dieser Zone werden durch Verbrennen eines Brennstoffs in Sauerstoff die Inertpartikel aufgeheizt. Die Reaktionswärme wird in die Reaktionszone durch die Mischung von Katalysator- und Inertteilchen eingebracht.

US 2,763,596 betrifft ein Verfahren zur Behandlung von Kohlenwasserstoffen in Gegenwart von Wasserstoff und in Gegenwart von festen Katalysatorteilchen, wobei die Aromatizität der Kohlenwasserstoffe steigt. Um die erforderliche Reaktionswärme in die Reaktionszone einzubringen, werden Wärmeüberträgerteilchen zum einen zwischen der Regenerationszone und der Reaktionszone und zum anderen zwischen Reaktionszone und Aufheizzone im Kreis gefahren. In der Regenerationszone werden Inertpartikel und Katalysatorpartikel mit Sauerstoff von den Kohlenstoffablagerungen unter Wärmefreisetzung regeneriert, in der Aufheizzone werden die Inertteilchen in Verbrennungsabgasen aufgeheizt.

Bei den aus dem Stand der Technik bekannten Verfahren werden die Katalysatorteilchen und die inerten Wärmeüberträgerteilchen durch die vielen notwendigen Transportvorgänge zwischen Reaktionszone, Regenerationszone und Aufheizzone starken mechanischen, chemischen und thermischen Belastungen ausgesetzt, die zu einer Verkürzung der Lebensdauer der Katalysatoren führen.

Über die im Stand der Technik bekannten Verfahren hinaus besteht somit Bedarf an weiteren, verbesserten Verfahren zur Durchführung von endothermen, heterogen katalysierten Reaktionen, insbesondere mit zeolithhaltigen Katalysatoren katalysierte Reaktionen.

Diese Aufgabe wird erfindungsgemäß gelöst durch ein Verfahren zur Durchführung von endothermen, heterogen katalysierten Reaktionen umfassend die Schritte
(a) Durchführen der Reaktion in einer Reaktionszone in Gegenwart einer Mischung enthaltend Katalysatorpartikel und inerte Wärmeüberträgerpartikel,
(b) Regenerieren der Katalysatorteilchen umfassend
   (b1) Überführen der Mischung enthaltend Katalysatorpartikel und ggf. inerte Wärmeüberträgerpartikel in eine Regenerationszone,
   (b2) Regenerieren der Katalysatorpartikel und ggf. der inerten Wärmeüberträgerpartikel in nicht-oxidativer Atmosphäre und
   (b3) Rückführen der regenerierten Katalysatorpartikel in die Reaktionszone, und
(c) Einbringen von Wärme in die Reaktionszone umfassend die Schritte
   (c1) Abtrennen der inerten Wärmeüberträgerpartikel von den Katalysatorpartikeln zwischen Schritt (a) und (b), während Schritt (b) oder nach Schritt (b),
   (c2) Überführen der abgetrennten inerten Wärmeüberträgerpartikel in eine Aufheizzone und
   (c3) Aufheizen der inerten Wärmeüberträgerpartikel und Rückführen der aufgeheizten inerten Wärmeüberträgerpartikel in die Reaktionszone.

Gemäß einer bevorzugten Ausführungsform handelt es sich bei der Reaktion in Schritt (a) um die nicht-oxidative Dehydroaromatisierung von C₁-C₄-Aliphaten in Gegenwart von Zeolith enthaltenden Katalysatorpartikeln.

Gemäß einer weiteren bevorzugten Ausführungsform werden die Katalysatorpartikel und gegebenenfalls die inerten Wärmeüberträgerpartikel in Schritt (b2) durch Zufuhr eines Wasserstoff enthaltenden Regenerationsgasstrom regeneriert.

Überraschenderweise hat sich gezeigt, dass die Abtrennung der Katalysatorpartikel von den inerten Wärmeüberträgerpartikeln vor dem Aufheizen der inerten Wärmeüberträgerpartikel in einem Riser oder in heißen Verbrennungsabgasen und die Regenerierung der Katalysatorpartikel in nicht-oxidativer Atmosphäre die Lebensdauer des Katalysators verlängert. Wie von den Erfindern herausgefunden wurde, nimmt beispielsweise bei der DHAM in Gegenwart auch kleiner Mengen Wasser bei den beim Aufheizen üblicherweise herrschenden Temperaturen von mehr als 700 °C die Reaktivität des zeolithhaltigen Katalysators, siehe Beispiel 1, ab. Dies geht mit einer Abnahme des Kristallinitätsgrades des im Katalysator enthaltenen Zeoliths einher. Bei der Verbrennung von Wasserstoffatomen enthaltenden Brennstoffen wie Methan mit Sauerstoff bzw. Luft entsteht Wasserdampf, der bei Aufheizen der Katalysatorpartikel durch die Verbrennung bzw. die dabei entstehenden Verbrennungsabgase den im Katalysator enthaltenen Zeolith irreversibel schädigt. Die Einkopplung der Reaktionswärme für die Dehydroaromatisierung von Methan durch externe Aufheizung der Katalysatorpartikel durch Verbrennen eines Brennstoffs wie Methan in einem Riser direkt in Gegenwart der aufzuheizenden Partikel, wie dies beispielsweise in US 2008/0249342 A1 beschrieben ist, kann daher den Katalysator irreversibel schädigen. Dieses Problem ließe sich umgehen, indem der Katalysator nicht durch den direkten Kontakt mit den Verbrennungsabgasen aufgeheizt wird, sondern mit den Verbrennungsabgasen ein wasserfreier Gasstrom (zum Beispiel Stickstoff oder Wasserstoff) aufgeheizt wird, der in direktem Kontakt mit dem Katalysator diesen aufheizt. Diese Verfahrensvariante ist jedoch technisch aufwendig (Wärmeübertragungsfläche, Inertgaskreislauf) und kostenintensiv. Zudem liegt der Gesamtenergiebedarf bei dieser Verfahrensvariante aufgrund der technischen Limitierungen, z.B. des eingesetzten Inertgasgebläses, höher als bei der direkten Beheizung.

Das erfindungsgemäße Verfahren besitzt somit den Vorteil, dass die Katalysatorpartikel nicht in direkten Kontakt mit den Verbrennungsabgasen kommen und daher nicht durch das darin enthaltene Wasser geschädigt werden. Da die Katalysatorpartikel lediglich zwischen Regenerationszone und Reaktionszone zirkulieren (und nicht auch noch in eine Aufheizzone geführt werden) und die inerten Wärmeüberträgerpartikel entweder gemeinsam mit den Katalysatorpartikeln in die Regenerationszone gelangen oder bereits vorher abgetrennt und in die Aufheizzone überführt werden, sind die erforderlichen Transportwege deutlich kürzer als bei den Verfahren aus dem Stand der Technik. Dies wirkt sich vorteilhaft auf die Katalysatorstandzeiten aus.

Bei Reaktionen, bei denen die Katalysatorpartikel durch Ablagerungen von Koks und/oder Kohlenstoff enthaltenden Ablagerungen deaktiviert werden, ist die Regenerierung durch Zufuhr eines Wasserstoff enthaltenden Regenerationsgasstroms besonders vorteilhaft, da hier der in den Ablagerungen enthaltende Kohlenstoff wieder in Methan umgewandelt und weiter verwertet werden kann, dies gilt insbesondere, falls Methan als Edukt bei der Reaktion in Schritt (a) eingesetzt wird. Werden die inerten Wärmeüberträger in der Regenerationszone von den Katalysatorpartikeln abgetrennt, können bei Einsatz eines Wasserstoff enthaltenden Regenerationsgasstroms auch gegebenenfalls auf den inerten Wärmeüberträgerpartikeln befindliche, kohlenstoffhaltige Ablagerungen wieder in Methan umgewandelt werden.

Ganz besondere Vorzüge weist eine Ausführungsform der vorliegenden Erfindung auf, bei der die Wärmeüberträgerpartikel und die Katalysatorpartikel in der Regenerationszone oder später voneinander abgetrennt werden und die Regeneration mittels eines Wasserstoff enthaltenden Regenerationsgases durchgeführt wird. In diesem Fall können die kohlenstoffhaltigen Ablagerungen auf beiden Partikelsorten in Kohlenwasserstoffe umgewandelt. Schließt sich dabei die Regenerationszone direkt an die Reaktionszone an, werden die Transportwege für die Katalysatorteilchen sehr kurz, und die mechanische Belastung der eingesetzten Katalysatorpartikel nochmals verringert.

Im Folgenden wird die Erfindung ausführlich erläutert.

"Heterogen katalysiert" bedeutet im Rahmen der Erfindung, dass mindestens ein Teil des oder der eingesetzten Katalysatoren, vorzugsweise die Gesamtmenge des oder der eingesetzten Katalysatoren, als Feststoff und das oder die eingesetzten Edukte gasförmig und/oder flüssig vorliegen.

Als "inerte Wärmeüberträgerpartikel" werden vorliegend Partikel verstanden, die die Reaktion in Schritt (a) nicht nachteilig beeinflussen, vorzugsweise an der in Schritt (a) durchgeführten Reaktion nicht teilnehmen und im Wesentlichen als Medium dienen, Wärme von außen in die Reaktionszone einzubringen.

In Bezug auf die Regenerierung bedeutet nicht-oxidativ im Rahmen der vorliegenden Erfindung, dass die aus der Reaktion in Schritt (a) stammenden Koksablagerungen auf dem Katalysator zu dessen Regenerierung nicht mittels Oxidationsmitteln wie Luft oder Sauerstoff in CO und/oder CO₂ überführt werden, sondern reduktiv entfernt werden. Insbesondere liegt die Konzentration an Oxidationsmitteln in dem zur Regenerierung in Schritt (b2) einzusetzenden Gemisch unterhalb von 5 Gew.-%, bevorzugt unterhalb von 1 Gew.-%, besonders bevorzugt unterhalb von 0,1 Gew.-%, ganz besonders bevorzugt frei von Oxidationsmitteln.

Nicht-oxidativ gemäß der vorliegenden Erfindung bedeutet in Bezug auf die Dehydroaromatisierung (DHAM) von C₁-C₄-Aliphaten, dass die Konzentration von Oxidationsmitteln wie Sauerstoff oder Stickoxiden im Eduktstrom E unterhalb von 5 Gew.-%, bevorzugt unterhalb von 1 Gew.-%, besonders bevorzugt unterhalb von 0,1 Gew.-% liegt. Ganz besonders bevorzugt ist das Gemisch frei von Sauerstoff. Ebenfalls besonders bevorzugt ist eine Konzentration an Oxidationsmitteln im Gemisch E, die gleich groß oder geringer ist als die Konzentration an Oxidationsmitteln in der Quelle, aus der die C₁-C₄-Aliphaten stammen.

In Schritt (a) des erfindungsgemäßen Verfahrens wird eine endotherme, heterogen katalysierte Reaktion in Gegenwart mindestens eines Katalysators, bevorzugt eines zeolithhaltigen Katalysators, durchgeführt. Bei dieser Reaktion kann es sich prinzipiell um alle endothermen, heterogen katalysierte Reaktionen handeln, bei denen die erforderliche Reaktionswärme direkt in die Reaktionszone zugeführt werden soll und die Katalysatorpartikel regelmäßig regeneriert werden müssen. Solche Reaktionen sind beispielsweise Dehydrierungen, insbesondere die nicht-oxidative Dehydroaromatisierung von Aliphaten, die dehydrierende Aromatisierung von Cycloaliphaten sowie Vergasungsreaktionen und Pyrolysen.

Erfindungsgemäß bevorzugt handelt es sich bei der in Schritt (a) durchgeführten Reaktion um die nicht-oxidative Dehydroaromatisierung von C₁-C₄-Aliphaten. Diese wird im Folgenden ausführlich beschrieben.

Bei der nicht-oxidativen Dehydroaromatisierung von C₁-C₄-Aliphaten (DHAM) wird ein Eduktstrom E enthaltend mindestens einen Aliphaten mit 1 bis 4 Kohlenstoffatomen in Gegenwart mindestens eines Katalysators unter Freisetzung von Wasserstoff zu Aromaten umgesetzt. Zu diesen Aliphaten gehören beispielsweise Methan, Ethan, Propan, n-Butan, i-Butan, Ethen, Propen, 1- und 2-Buten, Isobuten. In einer Ausführungsform der Erfindung enthält der Eduktstrom E mindestens 50 mol-%, bevorzugt mindestens 60 mol-%, besonders bevorzugt mindestens 70 mol-%, außerordentlich bevorzugt mindestens 80 mol-%, insbesondere mindestens 90 mol-% C₁-C₄-Aliphaten.

Unter den Aliphaten werden besonders bevorzugt die gesättigten Alkane verwendet, Eduktstrom E enthält dann mindestens 50 mol-%, bevorzugt mindestens 60 mol-%, besonders bevorzugt mindestens 70 mol-%, außerordentlich bevorzugt mindestens 80 mol-%, insbesondere mindestens 90 mol-% Alkane mit 1 bis 4 C-Atomen.

Unter den Alkanen sind Methan und Ethan bevorzugt, insbesondere Methan. Gemäß dieser Ausführungsform der vorliegenden Erfindung enthält der Eduktstrom E mindestens 50 mol-%, bevorzugt mindestens 60 mol-%, besonders bevorzugt mindestens 70 mol-% außerordentlich bevorzugt mindestens 80 mol-%, insbesondere mindestens 90 mol-% Methan.

Bevorzugt wird als Quelle der C₁-C₄-Aliphaten Erdgas eingesetzt. Die typische Zusammensetzung von Erdgas sieht folgendermaßen aus: 75 bis 99 mol-% Methan, 0,01 bis 15 mol-% Ethan, 0,01 bis 10 mol-% Propan, bis zu 6 mol-% Butan, bis zu 30 mol-% Kohlendioxid. bis zu 30 mol-% Schwefelwasserstoff, bis zu 15 mol-% Stickstoff und bis zu 5 mol-% Helium. Das Erdgas kann vor dem Einsatz in dem erfindungsgemäßen Verfahren nach dem Fachmann bekannten Methoden gereinigt und angereichert werden. Zur Reinigung gehört beispielsweise die Entfernung von gegebenenfalls im Erdgas vorhandenen Schwefelwasserstoff oder Kohlendioxid und weiterer, im anschließenden Verfahren unerwünschten Verbindungen.

Die in dem Eduktstrom E enthaltenen C₁-C₄-Aliphaten können auch aus anderen Quellen stammen, beispielsweise bei der Erdölraffination angefallen sein. Die C₁-C₄-Aliphaten können auch regenerativ (z.B. Biogas) oder synthetisch (z.B. Fischer-Tropsch-Synthese) hergestellt worden sein.

Falls als C₁-C₄-Aliphaten-Quelle Biogas verwendet wird, kann der Eduktstrom E zusätzlich noch Ammoniak, Spuren von niederen Alkoholen und weitere, für Biogas typische Beimischungen enthalten.

In einer weiteren Ausführungsform des erfindungsgemäßen Verfahrens kann als Eduktstrom E LPG (Liquid Petroleum Gas) eingesetzt werden. Gemäß einer weiteren Ausführungsform des erfindungsgemäßen Verfahrens kann als Eduktstrom E LNG (Liquified Natural Gas) eingesetzt werden.

Dem Eduktstrom E kann zusätzlich Wasserstoff, Kohlenmonoxid, Kohlendioxid, Stickstoff sowie ein oder mehrere Edelgase beigemischt werden. Vorzugsweise enthält der Eduktstrom E Wasserstoff, bevorzugt 0,1 bis 10 Vol.-% Wasserstoff, besonders bevorzugt 0,1 bis 5 Vol.-% Wasserstoff.

In der Reaktionszone findet die Umsetzung des Eduktstroms E unter nicht-oxidativen Bedingungen in Gegenwart eines teilchenförmigen Katalysators zu einem aromatische Kohlenwasserstoffe enthaltenden Produktstrom P statt. Bei der Dehydroaromatisierung reagieren die in dem Eduktstrom E enthaltenen C₁-C₄-Aliphaten unter Dehydrierung und Cyclisierung zu den entsprechenden Aromaten, wobei Wasserstoff freigesetzt wird. Die DHAM wird üblicherweise in Gegenwart geeigneter Katalysatoren durchgeführt. Dem Fachmann sind solche Katalysatoren wie auch Verfahren zu deren Herstellung bekannt. Üblicherweise enthalten die DHAM-Katalysatoren einen porösen Träger und mindestens ein darauf aufgebrachtes Metall. Als Träger wird üblicherweise eine kristalline oder amorphe anorganische Verbindung verwendet.

Erfindungsgemäß bevorzugt enthält der Katalysator mindestens ein Zeolith als Träger. Erfindungsgemäß besonders bevorzugt weist der in den Katalysatoren enthaltene Zeolith eine Struktur auf, die aus den Strukturtypen Pentasil und MWW ausgewählt ist und die insbesondere bevorzugt aus den Strukturtypen MFI, MEL und Mischstrukturen aus MFI und MEL und MWW ausgewählt ist. Ganz besonders bevorzugt wird ein Zeolith des Typs ZSM-5 oder MCM-22 eingesetzt. Die Bezeichnungen der Strukturtypen der Zeolithe entsprechen den Angaben in W. M. Meier, D. H. Olson und Ch. Baerlocher, "Atlas of Zeolithe Structure Types", Elsevier, 3. Auflage, Amsterdam 2001. Die Synthese der Zeolithe ist dem Fachmann bekannt und kann beispielsweise ausgehend von Alkalialuminat, Alkalisilikat und amorphem SiO₂ unter hydrothermalen Bedingungen durchgeführt werden. Hierbei kann über organische Templat-Moleküle, über die Temperatur und weitere experimentelle Parameter die Art der gebildeten Kanalsysteme im Zeolithen gesteuert werden.

Die Zeolithe können neben Al weitere Elemente wie Ga, B, Fe oder In enthalten.

Üblicherweise enthält der DHAM-Katalysator mindestens ein Metall. Üblicherweise wird das Metall ausgewählt aus den Gruppen 3 bis 12 des Periodensystems der Elemente (IUPAC). Erfindungsgemäß bevorzugt enthält der DHAM-Katalysator mindestens ein Element ausgewählt aus den Übergangsmetallen der Hauptgruppen 6 bis 11. Besonders bevorzugt enthält der DHAM-Katalysator Mo, W, Mn, Tc, Re, Fe, Ru, Os, Co, Rh, Ir, Ni, Pd, Pt, Cu, Ag, Au. Insbesondere enthält der DHAM-Katalysator mindestens ein Element ausgewählt aus der Gruppe Mo, W, Re, Fe, Ru, Os, Co, Rh, Ir, Ni, Pd, Pt, Cu. Ganz besonders bevorzugt enthält der DHAM-Katalysator mindestens ein Element ausgewählt aus der Gruppe Mo, W und Re.

Erfindungsgemäß ebenfalls bevorzugt enthält der DHAM-Katalysator mindestens ein Metall als Aktivkomponente und mindestens ein weiteres Metall als Dotierung. Die Aktivkomponente wird erfindungsgemäß ausgewählt aus Mo, W, Re, Ru, Os, Rh, Ir, Pd, Pt. Die Dotierung wird erfindungsgemäß ausgewählt aus der Gruppe Cr, Mn, Fe, Co, Ni, Cu, V, Zn, Zr und Ga, bevorzugt aus der Gruppe Fe, Co, Ni, Cu. Erfindungsgemäß kann der DHAM-Katalysator mehr als ein Metall als Aktivkomponente und mehr als ein Metall als Dotierung enthalten. Diese werden jeweils aus den für die Aktivkomponente und die Dotierung angegebenen Metallen ausgewählt.

Das mindestens eine Metall wird erfindungsgemäß nach den dem Fachmann bekannten Methoden nasschemisch oder trockenchemisch auf den Träger aufgebracht.

Erfindungsgemäß enthält der Katalysator 0,1 bis 20 Gew.-%, bevorzugt 0,2 bis 15 Gew.-%, besonders bevorzugt 0,5 bis 10 Gew.-%, jeweils bezogen auf das Gesamtgewicht des Katalysators des mindestens einen Metalls.

Erfindungsgemäß kann der Katalysator mindestens ein Metall aus der Gruppe der Aktivkomponente in Verbindung mit mindestens einem Metall ausgewählt aus der Gruppe der Dotierung enthalten. In diesem Fall liegt die Konzentration der Aktivkomponente bei 0,1 bis 20 Gew.-%, bevorzugt 0,2 bis 15 Gew.-%, besonders bevorzugt bei 0,5 bis 10 Gew.-%, jeweils bezogen auf das Gesamtgewicht des Katalysators.

Die Dotierung liegt in diesem Fall im Katalysator erfindungsgemäß in einer Konzentration von mindestens 0,1 Gew.-%, bevorzugt mindestens 0,2 Gew.-%, ganz besonders bevorzugt mindestens 0,5 Gew.-%, bezogen auf das Gesamtgewicht des Katalysators vor.

Gemäß einer weiteren bevorzugten Ausführungsform der vorliegenden Erfindung wird der Katalysator mit einem Bindemittel vermischt. Als Bindemittel eignen sich die üblichen, dem Fachmann bekannten Bindemittel wie Aluminiumoxid- und/oder Si-haltige Bindemittel. Besonders bevorzugt sind dabei Si-haltige Bindemittel; insbesondere eignen sich Tetraalkoxysilane, Polysiloxane und kolloidale SiO₂-Sole oder Mischungen aus den genannten Substanzen.

Wenn der erfindungsgemäße Katalysator ein Bindemittel oder Mischungen von Bindenmitteln enthält, liegt dies in einer Konzentration von 5 bis 80 Gew.-%, bezogen auf das Gesamtgewicht des Katalysators vor, bevorzugt von 10 bis 50 Gew.-%, besonders bevorzugt von 10 bis 30 Gew.-%.

Erfindungsgemäß erfolgt nach Zugabe des Bindemittels ein Formgebungsschritt, in dem die Katalysatormasse gemäß den dem Fachmann bekannten Verfahren zu Formkörpern verarbeitet werden. Als formgebende Verfahren sind dabei beispielsweise Versprühen einer den Träger bzw. die Katalysatormasse enthaltenden Suspension, Sprühtrocknung, Tablettieren, Verpressen im feuchten oder trockenen Zustand und Extrudieren zu nennen. Zwei oder mehrere dieser Verfahren können auch kombiniert werden. Für das Verformen können Hilfsmittel wie Porenbildner und Anteigungsmittel oder auch andere, dem Fachmann bekannte Zusatzstoffe eingesetzt werden.

Porenbildner und/oder Anteigungsmittel werden nach der Verformung bevorzugt durch mindestens einen geeigneten Trocknungs- und/oder Kalzinierungsschritt aus dem erhaltenen Formkörper entfernt. Die dazu erforderlichen Bedingungen können analog zu den vorstehend für Kalzinierung beschriebenen Parametern gewählt werden und sind dem Fachmann bekannt.

Die Geometrie der erfindungsgemäß erhältlichen Katalysatoren kann beispielsweise kugelförmig (hohl oder voll), zylindrisch (hohl oder voll), ring-, sattel-, stern-, bienenwaben- oder tablettenförmig sein. Weiterhin kommen Extrudate beispielsweise in Strang-, Trilob-, Quatrolob, Stern- oder Hohlzylinderform in Frage. Weiterhin kann die zu formende Katalysatormasse extrudiert, kalziniert und die so erhaltenen Extrudate gebrochen und zu Split oder Pulver verarbeitet werden. Der Split kann in verschiedene Siebfraktionen getrennt werden. Gemäß einer bevorzugten Ausführungsform der Erfindung wird der Katalysator als sprühgetrocknete Partikel, vorzugsweise Sprühpulver, eingesetzt. Dabei handelt es sich vorzugsweise um runde Teilchen. Die Katalysatorpartikel weisen vorzugsweise eine Größe von 10 bis 200 Mikrometer auf.

Vorzugsweise werden Katalysatorgeometrien eingesetzt, wie sie aus dem FCC-Verfahren (fuell catalytic cracking) bekannt sind.

Es kann von Vorteil sein, den zur Dehydroaromatisierung von C₁-C₄-Aliphaten verwendeten Katalysator vor der eigentlichen Reaktion zu aktivieren.

Diese Aktivierung kann mit einem C₁-C₄-Alkan, wie z.B. Methan, Ethan, Propan, Butan oder einem Gemisch hiervon, vorzugsweise Butan, erfolgen. Die Aktivierung wird bei einer Temperatur von 250 bis 850°C, vorzugsweise bei 350 bis 650°C, und einem Druck von 0,5 bis 5 bar, vorzugsweise bei 0,5 bis 4 bar, durchgeführt. Üblicherweise liegt die GHSV (Gas Hourly Space Velocity) bei der Aktivierung bei 100 bis 4000 h⁻¹, vorzugsweise bei 500 bis 2000 h⁻¹.

Es ist aber auch möglich eine Aktivierung durchzuführen, indem der Eduktstrom E das C₁-C₄-Alkan, oder ein Gemisch hiervon, per se schon enthält oder das C₁-C₄-Alkan, oder ein Gemisch hiervon, dem Eduktstrom E zugesetzt wird. Die Aktivierung wird bei einer Temperatur von 250 bis 650°C, vorzugsweise bei 350 bis 550°C, und einem Druck von 0,5 bis 5 bar, vorzugsweise bei 0,5 bis 2 bar, durchgeführt.

In einer weiteren Ausgestaltungsform ist es auch möglich zusätzlich zu dem C₁-C₄-Alkan noch Wasserstoff beizufügen.

Gemäß einer bevorzugten Ausführungsform der vorliegenden Erfindung wird der Katalysator mit einem H₂ enthaltenden Gasstrom aktiviert, der zusätzlich Inertgase wie N₂, He, Ne und Ar enthalten kann.

Erfindungsgemäß wird die Dehydroaromatisierung von C₁-C₄-Aliphaten bei Temperaturen von 400 bis 1000 °C, bevorzugt von 500 bis 900°C, besonders bevorzugt von 600 bis 800°C, insbesondere von 700 bis 800 °C, bei einem Druck von 0.5 bis 100 bar, bevorzugt bei 1 bis 30 bar, besonders bevorzugt bei 1 bis 10 bar, insbesondere 1 bis 5 bar, durchgeführt. Gemäß der vorliegenden Erfindung wird die Umsetzung bei einer GHSV (Gas Hourly Space Velocity, Volumenstrom Edukt/Volumen der Katalysatorschüttung) von 10 bis 10 000 h⁻¹, vorzugsweise von 20 bis 3000 h⁻¹ durchgeführt.

Die C₁-C₄-Aliphaten werden unter Freisetzung von H₂ zu Aromaten umgesetzt. Der dabei entstehende Produktstrom P enthält daher mindestens einen aromatischen Kohlenwasserstoff ausgewählt aus der Gruppe Benzol, Toluol, Ethylbenzol, Styrol, Xylol und Naphthalin. Besonders bevorzugt enthält er Benzol und Toluol. Weiterhin enthält der Produktstrom nicht umgesetzte C₁-C₄-Aliphaten entstandenen Wasserstoff und die im Eduktstrom E enthaltenen Inertgase wie N₂, He, Ne, Ar, dem Eduktstrom E zugesetzte Stoffe wie H₂ sowie bereits in E vorhandene Verunreinigungen.

Erfindungsgemäß wird die erforderliche Reaktionswärme mit Hilfe von inerten Wärmeüberträgerpartikeln in die Reaktionszone eingeführt. Die gemäß der Erfindung einzusetzenden inerten Wärmeüberträgerpartikel sollten wenig abrasiv sein, so dass durch sie so wenig Schäden wie möglich am Reaktor und den Transportrohren verursacht werden. Die Partikel sollten abriebsfest sein, um möglichst viele Wärmeüberträgerzyklen durchlaufen zu können. Weiterhin dürfen die inerten Wärmeüberträgerpartikel nicht zu spröde sein, um die auftretenden Stöße miteinander und gegenüber den Reaktor- bzw. Rohrleitungswänden unbeschadet zu überstehen. Zudem dürfen sie die in Schritt (a) durchgeführte Reaktion nicht nachteilig beeinflussen.

Prinzipiell können die inerten Wärmeüberträgerpartikel aus allen Materialien hergestellt werden, aus denen sich Partikel mit den vorstehend genannten Eigenschaften herstellen lassen. Die Wärmeüberträgerpartikel weisen bevorzugt eine abgerundete Form auf und besonders bevorzugt weisen sie eine im Wesentlichen kugelförmige Form auf. Das Material für die inerten Wärmeüberträgerpartikel kann beispielsweise aus der Gruppe bestehend aus Glas, Keramik, Siliziumcarbid, Metalloxiden wie Aluminiumoxid und Mischoxiden aus Siliziumdioxid mit Aluminiumoxid, Siliziumdioxid mit Magnesiumdioxid, Siliziumdioxid mit Thoriumdioxid, Siliziumdioxid mit Aluminiumoxid und Zirkoniumoxid, Zirkoniumoxid, Steatit und Sand ausgewählt werden, insbesondere Steatit, bevorzugt aus der Gruppe bestehend aus Glaskugeln, Keramikkugeln, Siliziumcarbidpartikeln, Al₂O₃-Partikel, Steatitpartikeln und Sand. Besonders bevorzugt sind die inerten Wärmeüberträgerpartikel abgerundete Steatitpartikel, insbesondere Steatitkugeln.

Das Gewichtsverhältnis von Wärmeüberträgerpartikeln und Katalysatorpartikeln in der Reaktionszone beträgt üblicherweise 2 : 1 bis 1 : 10, bevorzugt 1 : 1 bis 1 : 6. Das genaue Gewichtsverhältnis ist von den Fluidisationsbedingungen in der Reaktions- und Segregationszone sowie den Gas- und Partikeleigenschaften abhängig.

Der Massenstrom der Wärmeüberträgerpartikel ist bestimmt durch die Wärmemenge, die in die Reaktionszone eingetragen werden muss, um die Endothermie der Reaktion in Schritt (a) auszugleichen. Je größer die erforderliche Wärmemenge ist, desto höher wird entweder der Massenstrom an Wärmeüberträgerpartikeln oder deren Temperatur sein. Auch die bei der Regenerierung der Katalysatorpartikel gegebenenfalls entstehende Wärmemenge kann eine Rolle spielen. Bei der DHAM von C₁-C₄-Aliphaten beträgt das Gewichtsverhältnis von Wärmeüberträgerpartikel und Katalysatorpartikel in der Reaktionszone üblicherweise 2 : 1 bis 1 : 10, bevorzugt 1: 1 bis 1 : 6.

In Schritt (b) werden die Katalysatorpartikel regeneriert. Dazu wird in Schritt (b1) die Mischung enthaltend Katalysatorpartikel und gegebenenfalls inerte Wärmeüberträgerpartikel aus der Reaktionszone in eine Regenerationszone überführt. In der Regenerationszone werden die Katalysatorpartikel und gegebenenfalls die inerten Wärmeüberträgerpartikel in nicht-oxidativer Atmosphäre regeneriert (Schritt (b2)). Erfindungsgemäß bevorzugt wird die Regenerierung durch Zufuhr eines Wasserstoff enthaltenden Regenerationsgasstroms durchgeführt. Wenn der Katalysator in Schritt (a) durch Ablagerungen von Koks und weiteren Kohlenstoff enthaltenden Verbindungen deaktiviert wurde, werden diese Ablagerungen bei Regenerierung durch den im Regenerationsgasstrom enthaltenen Wasserstoff in Methan umgewandelt. Das gleiche geschieht auch zumindest teilweise mit den Ablagerungen auf den inerten Wärmeüberträgerpartikeln, falls diese nicht vor der Regenerierung von den Katalysatorpartikeln abgetrennt wurden. Die Umwandlung von Koksablagerungen in Methan ist exotherm und die Katalysatorpartikel und ggf. inerten Wäremüberträgerpartikel sowie der dabei entstehende, methanhaltige Gasstrom M können diese Wärme aufnehmen.

Die Regenerierung in Schritt (b2) wird üblicherweise bei Temperaturen von 600 °C bis 1000 °C und bevorzugt von 700 °C bis 900 °C durchgeführt. Die Drücke bei der Regenerierung liegen üblicherweise bei 1 bar bis 30 bar, bevorzugt bei 1 bar bis 15 bar und besonders bevorzugt bei 1 bar bis 10 bar. Bei der Regenerierung von kohlenstoffhaltigen Ablagerungen und Koks mit einem Wasserstoff enthaltenden Regenerationsgasstrom entsteht ein methanhaltiger Strom M, der neben dem entstandenen Methan weitere bei der Regenerierung entstandene Verbindungen, nicht umgesetzten Wasserstoff sowie schon im wasserstoffhaltigen Regenerationsgasstrom enthaltene Stoffe umfasst.

Üblicherweise beträgt die Konzentration des Wasserstoffs im Regenerationsgasstrom 20 bis 100 Vol.-%, bevorzugt 60 bis 100 Vol.-%.

Vorzugsweise wird die Konzentration des Wasserstoffs im Regenerationsgasstrom so bemessen, dass der bei Regenerierung kohlenstoffhaltiger Ablagerungen entstehende methanhaltige Gasstrom M bevorzugt höchstens 60 Vol.-%, besonders bevorzugt höchstens 20 Vol.-% und ganz besonders bevorzugt nur noch die Menge Wasserstoff enthält, die dem thermodynamischen Gleichgewicht unter diesen Bedingungen entspricht, d.h., dass der zugeführte Wasserstoff möglichst weitgehend und bevorzugt vollständig bei der Regenerierung in Schritt (b2) verbraucht wurde.

Wird in Schritt (a) eine Reaktion durchgeführt, bei der Methan als Edukt eingesetzt werden kann, insbesondere die DHAM, wird gemäß einer besonders bevorzugten Ausführungsform der Erfindung mindestens ein Teil des bei der Regenerierung entstandenen Methans der Reaktionszone in Schritt (a) zugeführt. Besonders bevorzugt werden mindestens 50 %, mehr bevorzugt mindestens 80 %, ganz besonders bevorzugt mindestens 90 % des Gasstroms M und insbesondere bevorzugt wird der gesamte Gasstrom M aus der Regenerationszone in die Reaktionszone überführt. Die vorstehend genannten Prozentangaben beziehen sich dabei auf das Volumen des Gasstroms M.

Gemäß einer bevorzugten Ausführungsform der vorliegenden Erfindung schließt sich die Regenerationszone direkt an die Reaktionszone an. Der Übergangsbereich zwischen der Reaktionszone und der Regenerationszone beträgt bevorzugt höchstens 25%, besonders bevorzugt höchstens 10 % und ganz besonders bevorzugt höchstens 5 % der Länge der Reaktionszone. Mit Länge der Reaktionszone ist die Ausdehnung des Reaktors parallel zur Hauptströmungsrichtung des Gases gemeint.

Besonders bevorzugt ist die Regenerationszone unterhalb der Reaktionszone angeordnet und weist höchstens den gleichen Querschnitt senkrecht zur Hauptströmungsrichtung der Partikel auf wie die Reaktionszone, bevorzugt einen mindestens 20 % geringeren Querschnitt.

An die Regenerationszone kann sich eine Strippzone anschließen. In der Strippzone werden die inerten Wärmeüberträgerpartikel von gegebenenfalls anhaftenden Katalysatorpartikeln, Edukten und/oder Produkten befreit. Das Strippen wird mit einem Strippgasstrom durchgeführt, der Inertgase wie Stickstoff und Argon enthalten kann, bevorzugt wird jedoch ein wasserstoffhaltiger Strippgasstrom eingesetzt.

Gemäß einer besonders bevorzugten Ausführungsform ist dabei die Regenerationszone direkt unterhalb der Reaktionszone und die Strippzone direkt unterhalb der Regenerationszone angeordnet. Die Strippzone weist bevorzugt höchstens den gleichen Querschnitt senkrecht zur Hauptströmungsrichtung der Partikel auf wie die Regenerationszone und besonders bevorzugt einen mindestens 20 % kleineren Querschnitt. Bevorzugt weist die Regenerationszone höchstens den gleichen Querschnitt senkrecht zur Hauptströmungsrichtung der Partikel auf wie die Reaktionszone, besonders bevorzugt einen mindestens 20% kleineren Querschnitt, wobei die Strippzone ihrerseits höchstens den gleichen, bevorzugt einen mindestens 20 % kleineren Querschnitt senkrecht zur Hauptströmungsrichtung der Partikel als die Regenerationszone aufweist.

Ist die Regenerationszone direkt unterhalb der Reaktionszone angeordnet, werden die Edukte für die in Schritt (a) durchzuführende Reaktion bevorzugt im unteren Teil der Reaktionszone zugeführt, besonders bevorzugt im unteren Drittel und ganz besonders bevorzugt im untersten Viertel der Reaktionszone zugeführt. Die bei der Reaktion entstandenen gasförmigen und/oder flüssigen Produkte werden dabei üblicherweise aus dem oberen Teil der Reaktionszone, bevorzugt aus dem oberen Drittel und besonders bevorzugt aus dem obersten Viertel der Reaktionszone aus der Reaktionszone ausgetragen und insbesondere über Kopf abgezogen, dies ist insbesondere zweckdienlich, wenn es sich um gasförmige Produkte handelt.

Bei Regenerierung der Katalysatorpartikel durch Zufuhr eines Wasserstoff enthaltenden Regenerationsgasstromes wird dieser in Schritt (b2) bei Anordnung der Regenerationszone unterhalb der Reaktionszone dem unteren Teil, bevorzugt dem unteren Drittel und besonders bevorzugt dem untersten Viertel der Regenerationszone zugeführt.

Nach Regenerieren der Katalysatorpartikel werden die regenerierten Katalysatorpartikel in die Reaktionszone zurückgeführt. Dabei können die Katalysatorpartikel außerhalb oder innerhalb des Reaktors zurückgeführt werden.

Wenn sich die Regenerationszone direkt an die Reaktionszone anschließt, ist erfindungsgemäß bevorzugt, dass das Überführen des verkokten Katalysators und ggf. der inerten Wärmeüberträgerpartikel aus der Reaktionszone in die Regenerationszone direkt, d.h. ohne Umleitung in dem Bereich erfolgt, in dem die beiden Reaktionszonen räumlich aneinander grenzen. Wird in Schritt (a) eine Reaktion durchgeführt, bei der Methan als Edukt eingesetzt werden kann, insbesondere die DHAM, erfolgt auch das Überführen mindestens eines Teils des bei der Regenerierung entstehenden Gasstroms M in Schritt (b2) aus der Regenerationszone in die Reaktionszone bevorzugt direkt. Die mittlere Strömungsrichtung des Gasstroms M ist dabei erfindungsgemäß gegenläufig zur mittleren Strömungsrichtung der verkokten Katalysatorpartikel. Bei äußerer Rückführung wird der regenerierte Katalysator bei Anordnung der Regenerationszone unterhalb der Reaktionszone in Schritt (b3) vorzugsweise in den oberen Teil der Reaktionszone, mehr bevorzugt in das obere Drittel und ganz besonders bevorzugt in das oberste Viertel der Reaktionszone zurückgeführt. Insbesondere bevorzugt werden die Katalysatorpartikel von oben in die Reaktionszone zurückgeführt.

Bei der gemäß Schritt (a) durchzuführenden Reaktion als auch beim Regenerieren des deaktivierten Katalysators in nicht oxidativer Atmosphäre gemäß Schritt (b2) können die Katalysatorpartikel und die inerten Wärmeüberträgerpartikel als Wirbelschicht, Wanderbett oder Fließbett in den entsprechenden, dafür geeigneten Reaktortypen vorliegen. Erfindungsgemäß bevorzugt liegen die Katalysatorpartikel und die inerten Wärmeüberträgerpartikel in der Reaktionszone, in der Regenerationszone oder in beiden Zonen als fluidisiertes Bett vor, besonders bevorzugt werden die Reaktionszone und die Regenerationszone als gemeinsames, zoniertes Wirbelbett betrieben.

Erfindungsgemäß bevorzugt werden die Betriebsparameter, Reaktorausgestaltung und Reaktorabmessungen so gewählt, dass im Wesentlichen keine Gasrückvermischung von der Reaktionszone in die Regenerationszone auftritt, auch wenn sich die Regenerationszone direkt an die Reaktionszone anschließt, um nach Möglichkeit einen Eintrag von Edukten aus der Reaktionszone in die Regenerationszone zu verhindern. Es könnte sich bei bestimmten Reaktionen hinsichtlich der reduktiven Regenerierung des Katalysators negativ auswirken. Beispielsweise entsteht bei der Regenerierung des bei der DHAM von C₁-C₄-Aliphaten deaktivierten Katalysators mit Wasserstoff Methan, daher wirkt sich ein Eintrag dieser Aliphaten, insbesondere von Methan negativ auf das Reaktionsgleichgewicht der Regenerierung aus.

Besonders bevorzugt wird die Regenerationszone als Wirbelschicht betrieben, wobei im Wesentlichen keine interne Durchmischung der Gasphase auftritt. Die interne Durchmischung soll möglichst unterbunden werden, um eine Rückvermischung des Edukte enthaltenden Stroms aus der Reaktionszone in die Regenerationszone zu vermeiden bzw. zumindest zu verringern und so in der Regenerationszone eine Atmosphäre zu gewährleisten, die möglichst rein an Reduktionsmittel und/oder Inertgas ist. Bei der Regenerierung der Katalysatorpartikel mit Wasserstoff soll auf diese Weise eine möglichst reine Wasserstoff-Atmosphäre bereitgestellt werden. Insbesondere bei der DHAM von C₁-C₄-Aliphaten führt eine möglichst methanarme Gasphase in der an die Reaktionszone direkt angeschlossene Regenerationszone zur besseren Regeneration der zu regenerierenden Katalysatorpartikel.

Die Bedingungen für den Betrieb des Katalysatorbetts enthaltend Katalysatorpartikel und gegebenenfalls inerte Wärmeüberträgerpartikel mit möglichst geringer interner Durchmischung sind dem Fachmann bekannt. Hinweise für die Auswahl der Parameter/Betriebsbedingungen können beispielsweise in D. Kunii, O. Levenspiel "Fluidization Enginneering Second Edition, Boston, Kapitel 9, Seiten 211 bis 215 und Kapitel 10, Seiten 237 bis 243, gefunden werden.

Eine weitere Möglichkeit, die interne Durchmischung in der Regenerationszone zu vermindern, ist der Einbau bzw. die Anordnung von Vorrichtungen, die die interne Durchmischung behindern. Diese Vorrichtungen können beispielsweise Lochbleche, strukturierte Packungen, Leitbleche und weitere, dem Fachmann bekannte Einbauten sein. Gemäß einer bevorzugten Ausführungsform ist mindestens eine derartige Vorrichtung in der Regenerationszone angeordnet. Das Ausmaß der internen Vermischung kann beispielsweise anhand der vertikalen Dispersionskoeffizienten bestimmt werden.

Bei Durchführung einer DHAM in Schritt (a) wird vorzugsweise weniger als 10 mol-% C₁-C₄-Aliphat, insbesondere Methan, bezogen auf den Regenerationsgasstrom durch Rückvermischung von der Reaktionszone in die Regenerationszone eingetragen.

Erfindungsgemäß bevorzugt ist die Reaktionszone von der Regenerationszone durch mindestens eine für die Reaktionsströme und die Katalysatorpartikel und inerten Wärmeüberträgerpartikel durchlässige, im Übergangsbereich zwischen der Reaktionszone und der Regenerationszone angeordneten Vorrichtung getrennt. Bei diesen Vorrichtungen kann es sich um Lochbleche, Leitbleche, strukturierte Packungen und weitere dem Fachmann bekannte, für diesen Zweck geeignete Einbauten handeln, wie beispielsweise im Handbook of Fluidization and Fluid-Particle Systems, New York, 2003, Editor W. Yang, Kapitel 7, Seiten 134 bis 195 beschrieben. Durch diese Vorrichtungen kann die Rückvermischung der Katalysatorteilchen und der Reaktionsgase zwischen diesen beiden Zonen beeinflusst werden. Als Reaktionsgase wird im Rahmen der Erfindung die Gesamtheit der in der Reaktionszone und der Regenerationszone beteiligten Gasströme bezeichnet, also die Gasströme E, P, ggf. wasserstoffhaltige Regenerationsgasstrom und ggf. Strippgasstrom

Die Reaktionszone wird erfindungsgemäß bevorzugt als blasenbildendes oder turbulentes Wirbelbett betrieben, üblicherweise bei Leerrohrgasgeschwindigkeiten von 10 bis 100 cm/s.

Erfindungsgemäß bevorzugt werden die Katalysatorpartikel und die inerten Wärmeüberträgerpartikel einerseits und die verschiedenen Ströme (Edukte, zur Regenerierung der Katalysatorpartikel eingesetzter Strom, Strippgas) andererseits im Gegenstrom geführt. Ist die Regenerationszone gemäß der vorstehend beschriebenen bevorzugten Ausführungsform direkt unterhalb der Reaktionszone angeordnet, bedeutet das, dass die inerten Wärmeüberträgerpartikel im Mittel von oben nach unten strömen und die Eduktströme, Produktströme, zur Regenerierung der Katalysatorpartikel eingesetzte Ströme und die Strippgasströme eine mittlere Strömungsrichtung von unten nach oben aufweisen. Die Katalysatorpartikel bewegen sich aufgrund der internen Feststoffzirkulation zwischen der Reaktions- und der Regenerationszone. Dabei bewegen sich die in der Reaktionszone verkokten Partikel im Mittel von oben nach unten während die in der Regenerationszone regenerierten Partikel sich im Mittel von unten oben bewegen.

Während der Reaktion in Schritt (a) liegen die inerten Wärmeüberträgerpartikel mit den Katalysatorpartikeln gemischt vor. In Schritt (c1) werden die inerten Wärmeüberträgerpartikel von den Katalysatorpartikel abgetrennt. Dabei ist erfindungswesentlich, dass die Abtrennung so durchgeführt wird, dass im Wesentlichen lediglich die inerten Wärmeüberträgerpartikel in die Aufheizzone gelangen, die Katalysatorpartikel jedoch in nicht-oxidativer Atmosphäre regeneriert werden. Das Abtrennen der inerten Wärmeüberträgerpartikel von den Katalysatorpartikeln kann nach verschiedenen Methoden erfolgen. Dazu eignen sich verschiedene Prozesse wie beispielsweise Segregation, Sichtung, magnetische Abtrennung, Siebung, elektrostatische Abtrennung oder jede andere Möglichkeit der Trennung verschiedenartiger Partikel. Segregation, Sichtung und Siebung beruhen beispielsweise auf unterschiedlichen Größen und Dichten der Katalysatorpartikel und der inerten Wärmeüberträgerpartikel, die magnetische Abtrennung erfolgt aufgrund unterschiedlicher magnetischer Eigenschaften der zu trennenden Partikel.

Erfindungsgemäß bevorzugt werden die inerten Wärmeüberträgerpartikel von den Katalysatorpartikeln durch Segregation abgetrennt. Dazu müssen die inerten Wärmeüberträgerpartikel und die Katalysatorpartikel unterschiedliche Fluidisierungseigenschaften aufweisen, so dass sie bei unterschiedlichen Gasgeschwindigkeiten fluidisiert werden. Als allgemeine Daumenregel gilt, dass größere Teilchen mit einer höheren Dichte in einem Wirbelbett dazu neigen, sich im unteren Teil zu sammeln, wohingegen kleinere Partikel mit einer niedrigeren Dichte deutlich leichter und bei niedrigeren Gasflüssen fluidisiert werden und daher bei entsprechender Wahl der Flussparameter und Teilcheneigenschaften nach oben wandern.

Wenn die inerten Wärmeüberträgerpartikel größer sind und eine höhere Dichte aufweisen als die Katalysatorpartikel, wird eine Trennung der beiden Partikelsorten dadurch erreicht, dass ein Gasstrom mit einer Flussrate durch das Partikelgemisch geleitet wird, die hoch genug ist, um die größten Katalysatorpartikel zu fluidisieren, jedoch nicht ausreicht, um die inerten Wärmeüberträgerpartikel zu fluidisieren. Nach Trennen der inerten Wärmeüberträgerpartikel und Katalysatorpartikel kann der Gasstrom, der durch die inerten Wärmeüberträgerpartikel hindurchströmt, so angepasst werden, dass die inerten Wärmeüberträgerpartikel wieder fluidisiert werden und leicht in die Aufheizzone transportiert werden können.

Die Trennung der inerten Wärmeüberträgerpartikel und der Katalysatorpartikel erfolgt dabei möglichst vollständig, bevorzugt enthalten die abgetrennten Wärmepartikel höchstens 0,1 Gew.-% Katalysatorpartikel, bezogen auf die Gesamtmenge der abgetrennten Partikel (abgetrennte Wärmeüberträgerpartikel und mit diesen abgetrennte Katalysatorpartikel).

Bevorzugt haben die Inertpartikel etwa die doppelte Partikeldichte und die 10-fache Größe

Die Abtrennung der inerten Wärmeüberträgerpartikel von den Katalysatorpartikeln in Schritt (c1) kann zwischen Schritt (a) und (b), während Schritt (b) oder nach Schritt (b) durchgeführt werden. Bevorzugt wird die Abtrennung der inerten Wärmeüberträgerpartikel während oder nach Schritt (b) durchgeführt. Besonders bevorzugt wird die Abtrennung der inerten Wärmeüberträgerpartikel von den Katalysatorpartikeln in Schritt (b2) während der Regenerierung der Katalysatorpartikel in der Regenerationszone durchgeführt. Ebenfalls besonders bevorzugt ist die Abtrennung der inerten Wärmeüberträgerpartikel in der Strippzone, falls eine solche vorhanden ist.

Sind die Regenerationszone und die ggf. vorhandene Strippzone direkt im Anschluss an die Reaktionszone direkt unterhalb von dieser angeordnet und werden die inerten Wärmeüberträgerpartikel von den Katalysatorpartikeln in der Regenerationszone oder der ggf. vorhandenen Strippzone abgetrennt, erfolgt dies gemäß einer bevorzugten Ausführungsform durch Segregation. Dabei weisen die inerten Wärmeüberträgerpartikel und die Katalysatorpartikel unterschiedliche Fluidisierungseigenschaften auf und werden voneinander getrennt, indem durch entsprechendes Einstellen des Regenerationsgasstroms in der Regenerationszone bzw. des Strippgasstroms in der Strippzone die Katalysatorpartikel und die inerten Wärmeüberträgerpartikel unterschiedlich fluidisiert werden und entmischen. Besonders gut eignet sich dabei der Wasserstoff enthaltender Regenerationsgasstrom bzw. ein Wasserstoff enthaltender Strippgasstrom, der anschließend als Regenerationsgasstrom dienen kann.

Nach dem Abtrennen der inerten Wärmeüberträgerpartikel von den Katalysatorpartikeln werden die abgetrennten inerten Wärmeüberträgerpartikel in eine Aufheizzone überführt (Schritt (c2)).

In der Aufheizzone werden die inerten Wärmeüberträgerpartikel aufgeheizt und anschließend die aufgeheizten inerten Wärmeüberträgerpartikel in die Reaktionszone zurückgeführt (Schritt (c3)). Die inerten Wärmeüberträgerpartikel werden in der Aufheizzone durch Kontakt mit heißem Inertgas, Kontakt mit heißem Verbrennungsabgas, direkte Verbrennung mindestens eines Brennstoffs wie beispielsweise das bei der nicht-oxidativen Dehydroaromatisierung von C₁-C₄-Aliphaten eingesetzte Edukt, Abtrennen von Ablagerungen auf den Wärmeüberträgerpartikeln, Kontakt mit heißen Oberflächen, Einwirken von elektromagnetischen Wellen, elektrisch und/oder durch Induktion erhitzt. Bevorzugt werden die abgetrennten inerten Wärmeüberträgerpartikel in Schritt (c3) durch Kontakt mit heißem Verbrennungsabgas, direkter Verbrennung mindestens eines Brennstoffs und/oder Abbrennen von Ablagerungen auf den Wärmeüberträgerpartikeln erhitzt.

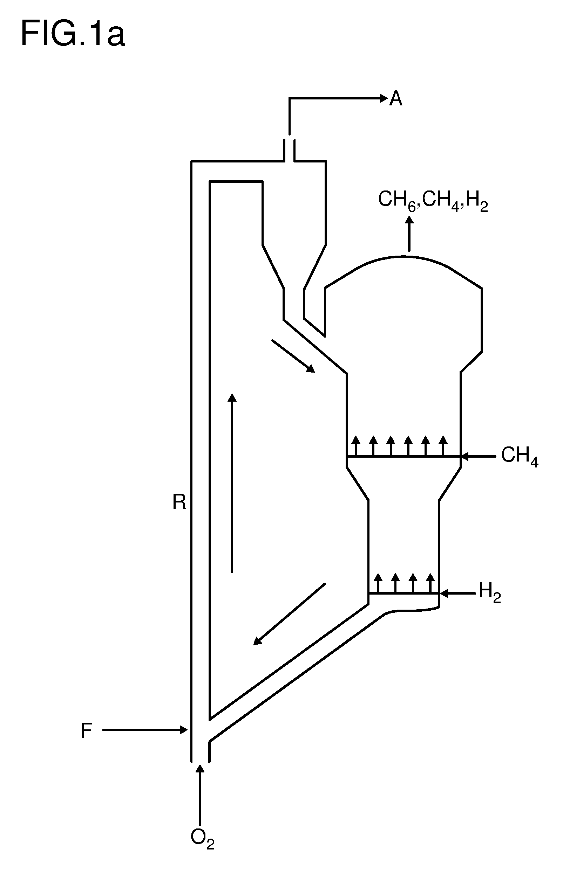

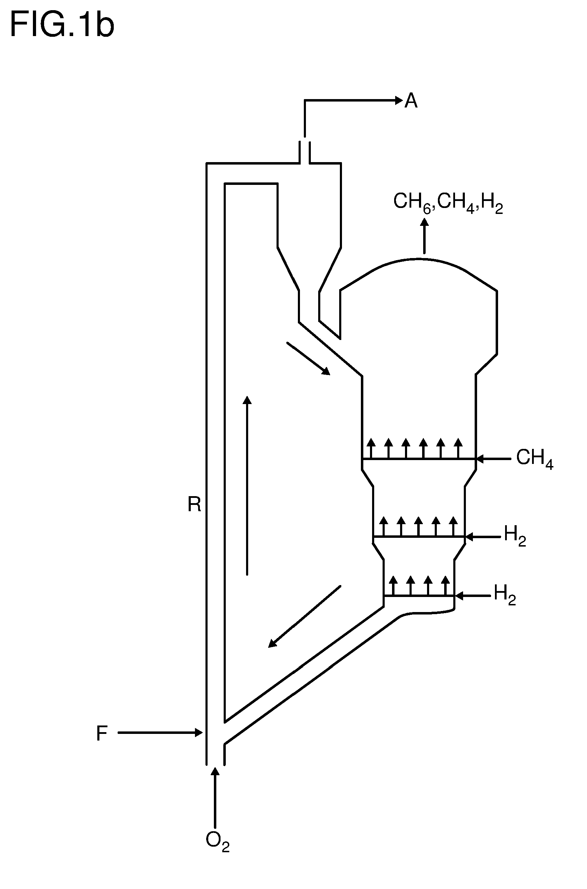

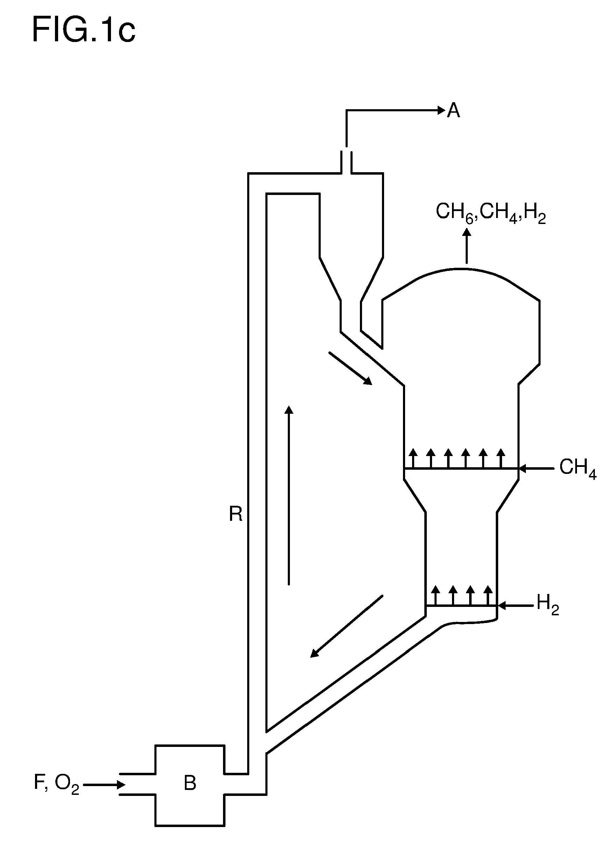

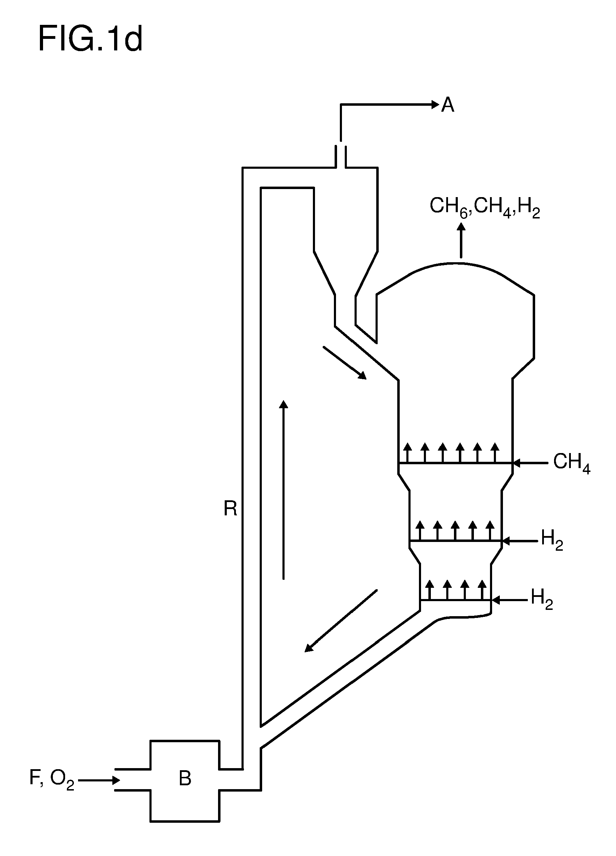

Vier für die Durchführung es erfindungsgemäßen Verfahrens besonders geeignete Reaktorformen sind in Figur 1 anhand der nicht-oxidativen Aromatisierung von Methan unter Regenerierung der deaktivierten Katalysatorpartikel mit einem Wasserstoff enthaltenden Regenerationsgasstrom dargestellt (Figur 1(a) bis (d)).

F bedeutet dabei Brennstoff, B bezeichnet eine Verbrennungseinrichtung und A das bei der Verbrennung entstehende Abgas. Gemäß den in den Figuren 1(a) und (b) gezeigten Reaktorschemata wird der Brennstoff (F) und Sauerstoff, beispielsweise Luft, direkt in Gegenwart der aufzuheizenden inerten Wärmeüberträgerpartikel verbrannt. Gemäß den in Figur 1(c) und (d) dargestellten Reaktionsschemata wird der Brennstoff (F) in einer Brennvorrichtung (B) mit Sauerstoff verbrannt und die dabei entstehenden heißen Verbrennungsabgase über die inerten Wärmeüberträgerpartikel geleitet.

Bei der in Figur 1(a) bis (d) ist jeweils die bevorzugte Ausführungsform der Erfindung dargestellt, in der sich direkt unterhalb an die Reaktionszone die Regenerationszone anschließt. In den unteren Teil der Reaktionszone wird jeweils CH₄ geleitet, in den unteren Teil der Regenerationszone wird jeweils Wasserstoff geleitet. Bei den in Figur 1(b) und (d) dargestellten Ausführungsformen ist direkt unterhalb an die Regenerationszone noch eine Strippzone angeschlossen, wobei hier als Strippgas ein Wasserstoff enthaltendes Gasgemisch eingesetzt wird. Gemäß den in Figur (a) und (c) dargestellten Ausführungsformen werden die inerten Wärmeüberträgerpartikel in der Regenerationszone von den Katalysatorpartikeln getrennt und über die nach unten führende Rohrleitung in einen Riser (R) überführt, wo sie aufgeheizt werden. Die Wärmeüberträgerpartikel werden dabei aufsteigend wieder der Reaktionszone zugeführt. Gemäß der in Figur 1(b) und (d) dargestellten Ausführungsformen werden die inerten Wärmeüberträgerpartikel in der Strippzone von den Katalysatorpartikeln abgetrennt und in gleicher Weise wie vorstehend beschrieben im Riser (R) aufgeheizt, nach oben geführt und von oben wieder der Reaktionszone zugeführt.

Im Folgenden wird die Erfindung anhand von Beispielen erläutert.

### Beispiel 1 Einfluss von Wasserdampf im Reaktionsgas der DHAM

Ca. 1,6 g des Katalysators (6% Mo, 1% Ni auf einem H-ZSM-5 Träger mit SiO₂:Al₂O₃ Verhältnis von 25) wurden im Reaktorrohr (Innendurchmesser = 4 mm) unter Heliumatmosphäre auf 500 °C erwärmt. Bei dieser Temperatur wurde Methan zugeschaltet und der Katalysator 30 min bei dieser Temperatur gehalten, bevor er unter Methan, das 10 Vol.-% Helium enthielt, auf die Reaktionstemperatur von 700 °C gebracht wurde. Der Katalysator wurde dann für ca. 35 h bei 700 °C, 1 bar, 10 Vol.-% He in Methan und einer GHSV von 500 h⁻¹ betrieben. Während der Reaktion wurde das Eingangsgas 180 Minuten lang durch einen Sättiger geleitet und hierdurch der Eingangsgasmischung 2,8 Vol.-% Wasserdampf zugesetzt. Nach der Reaktion wurde von den ausgebauten Katalysatoren mittels Röntgenstreuung (XRD) der Kristallinitätsgrad des ZSM-5 Zeolithträgers bestimmt.

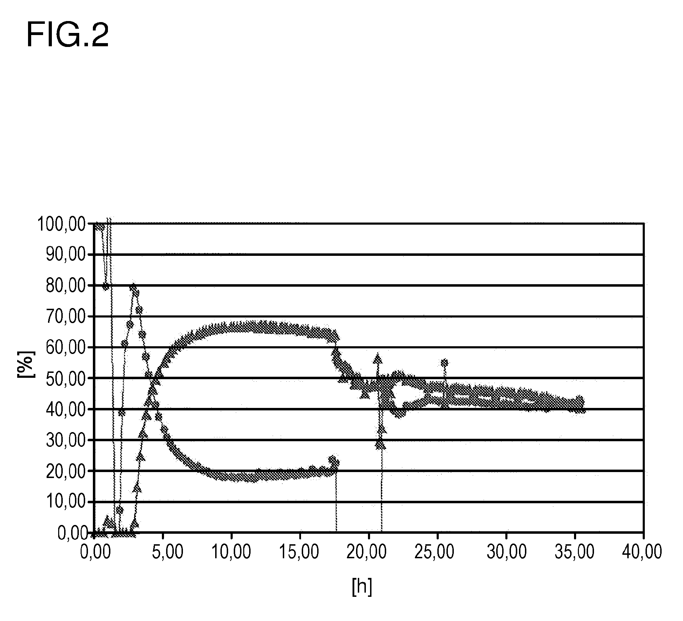

Die Benzolselektivitäten und die gemessenen Kristallinitätsgrade sind in Tabelle 1 dargestellt. Als Zeitpunkt 0 min ist in Tabelle 1 der Beginn der Wasserdampfzudosierung bezeichnet, diese wurde 17,5 Stunden nach Reaktionsbeginn gestartet. Die Benzolselektivität S_{B} (gefüllte Dreiecke) und die Koksselektivität (gefüllte Kreise) in Abhängigkeit von der Reaktionszeit t sind in Figur 2 gezeigt.

**Tabelle 1**

| Reaktionszeit t [h] | Dosierungszeit des Wasserdampfs | Kristallinitätsgrad von ZSM-5 des Ausgebauten Katalysators | S_{B} |
|---|---|---|---|
| 17,5 | 0 min | 71% | 68% |
| 18 | 30 min | 68% | 66% |
| 18,7 | 40 min | 67% | 64% |
| 20,5 | 180 min | 54% | 51% |

| | | | |
|---|---|---|---|
| S_{B}: Benzolselektivität, Menge des zu Benzol umgesetzten Methans bezogen auf die Menge umgesetztes Methan | | | |

Die Ergebnisse der Röntgenstreuung zeigen, dass bei Anwesenheit von Wasserdampf im Reaktionsgas unter Reaktionsbedingungen der Zeolithträger geschädigt wird, was zu einer irreversiblen Verringerung der Benzolselektivität des Katalysators führt. Je länger die Wasserexposition des Katalysators andauert, desto mehr nimmt dabei die Benzolselektivität des Katalysators ab. In einem kontinuierlichen Prozess muss daher die Anwesenheit von Wasserdampf im Reaktionsgas vermieden werden.

### Beispiel 2

Für Beispiel 2 wurde ein Glasrohr mit einem Innendurchmesser von 40 mm und einer Gesamthöhe von etwa 2,5 m verwendet. Am Boden der Anlage befand sich eine Glasfritte, über die die Gaseinleitung und Gasverteilung erfolgte. Am unteren Ende der Anlage war seitlich ein schräg nach unten verlaufender Stutzen angebracht, über den eine Feststoffprobe entnommen werden konnte. Als Gas zur Fluidisierung wurde Stickstoff eingesetzt. Um während der Versuche elektrostatische Effekte zu minimierem, wurde der Stickstoff über eine Waschflasche bei Raumtemperatur geleitet, um ihn mit etwas Wasser anzufeuchten.

Als Modellsubstanz für die Katalysatorpartikel diente ein Aluminiumoxidpulver (Puralox SCCa 57/170, Fa. Sasol), das mit einer wässrigen Natriumchloridlösung mit einer Konzentration von 6 mol/l getränkt wurde. Die Menge der Natriumchloridlösung entsprach etwa 40 Gew.-% der Partikelmenge.

Zur Bestimmung des Aluminiumoxidgehalts in den ausgeschleusten Partikelproben dienten Leitfähigkeitsmessungen. Dazu wurden zu 10 g der Feststoffprobe 100 ml voll entsalztes Wasser gegeben und diese Mischung dann ca. 2 Minuten gerührt. Die Inertpartikel setzten sich dabei sofort am Boden ab, das Aluminiulmoxid befand sich noch in der Schwebe. Gemessen wurde die Leitfähigkeit im Wasser oberhalb der Partikel, nachdem diese sich nahezu vollständig abgesetzt hatten.

### Beispiel 2a: Leitfähigkeit der reinen Stoffe

| | |
|---|---|
| Glaskugeln, gewaschen und getrocknet (10 g in 100 ml Wasser): | 6-8 µS/cm |
| frisch getränktes Puralox (1 g in 100 ml Wasser) | ca. 1700 µS/cm |
| ungetränktes Puralox (1 g bzw. 10 g in 100 ml Wasser) | 1 µS/cm |
| reines VE-Wasser | 4 µS/cm |
| Magnesiumsilikat: | 13 µS/cm |

### Beispiel 2b Bestimmung der Trenneigenschaften

Zur Bestimmung der Trenneigenschaften wurden unterschiedliche Inertpartikel eingesetzt. Dabei handelte es sich um Steatitteilchen mit zwei unterschiedlichen Größenbereichen und Glaskugeln. Die Eigenschaften der verwendeten Inertpartikel und der Katalysatorpartikel sind in Tabelle 2 zusammengefasst. Die Partikelgrößenverteilung wurde jeweils durch Siebanalyse bestimmt.

**Tabelle 2**

| | Inertpartikel 1 | Inertpartikel 2 | Inertpartikel 3 | Katalysator |
|---|---|---|---|---|
| Material | Steatit | Glas | Steatit | Al₂O₃ |
| d_{p,50} [µm] | 240 | 500 | 750 | 50 |
| ρ [kg/m³] | 1422 | 1438 | 1563 | 800 |
| U_{mf} [cm/s] | 14,3 | 28 | 41,1 | 0,3 |
| u₀ [cm/s] | 10 | 20 | 30 | |

| | | | | |
|---|---|---|---|---|
| d_{p,50}: mittlerer Partikeldurchmesser p: Schüttdichte u_{mf}: minimale Fluidisationsgeschwindigkeit u₀: Im Versuch eingestellte Lehrrohrgasgeschwindigkeit | | | | |

Der Austrag an Katalysatorpartikeln wurde bestimmt, indem die Aluminiumoxidpartikel als Modellsubstanz vorgelegt (Schütthöhe ca. 350mm) und entsprechend den in Tabelle 2 genannten Leerrohrgasgeschwindigkeiten mit Stickstoff fluidisiert wurden. Anschließend wurden die Inertpartikel kontinuierlich über eine Dosierschnecke mit einer Dosierrate von 120g/min von oben zugegeben. Nachdem sich im unteren Bereich des Wirbelbettes Inertpartikel angereichert hatten, wurden aus diesem Bereich kontinuierlich der gleiche Massenstrom an Inertpartikeln abgezogen wie er oben zudosiert wurde. Nachdem die Anlage für ca. 5min stationär betrieben lief, wurde aus dem abgezogenen Strom eine Probe entnommen und nach der oben beschriebenen Methode analysiert.

Die Ergebnisse für die drei untersuchten Inertpartikel sind in Tabelle 3 dargestellt.

**Tabelle 3**

| | Inertpartikel 1 | Inertpartikel 2 | Inertpartikel 3 |
|---|---|---|---|
| U_{mf', In},/u_{mf, Kat} | 48 | 93 | 137 |
| m_{Kat} | 0,33% | 0,04% | 0% |

| | | | |
|---|---|---|---|
| u_{mf, In} In: minimale Fluidisationsgeschwindigkeit der Inertteilchen u_{mf, Kat} Kat: minimale Fluidisationsgeschwindigkeit der Katalysatorpartikel m_{Kat}: Masse an Katalysatorpartikeln bezogen auf die Gesamtmasse der entnommenen Partikelprobe in % | | | |

## Patentansprüche

1. Verfahren zur Durchführung von endothermen, heterogen katalysierten Reaktionen umfassend die Schritte
(a) Durchführen der Reaktion in einer Reaktionszone in Gegenwart einer Mischung enthaltend Katalysatorpartikel und inerte Wärmeüberträgerpartikel,
(b) Regenerieren der Katalysatorpartikel umfassend
(b1) Überführen der Mischung enthaltend Katalysatorpartikel und ggf. inerte Wärmeüberträgerpartikel in eine Regenerationszone,
(b2) Regenerieren der Katalysatorpartikel und ggf. der inerten Wärmeüberträgerpartikel in nicht-oxidativer Atmosphäre und
(b3) Rückführen der regenerierten Katalysatorpartikel in die Reaktionszone, und
(c) Einbringen von Wärme in die Reaktionszone umfassend die Schritte
(c1) Abtrennen der inerten Wärmeüberträgerpartikel von den Katalysatorpartikeln zwischen Schritt (a) und (b), während Schritt (b) oder nach Schritt (b),
(c2) Überführen der abgetrennten inerten Wärmeüberträgerpartikel in eine Aufheizzone und
(c3) Aufheizen der inerten Wärmeüberträgerpartikel und Rückführen der aufgeheizten inerten Wärmeüberträgerpartikel in die Reaktionszone,
**dadurch gekennzeichnet, dass** es sich bei der Reaktion in Schritt (a) um die nicht-oxidative Dehydroaromatisierung von C₁- bis C₄-Aliphaten handelt.

2. Verfahren nach Anspruch 1, **dadurch gekennzeichnet, dass** die Katalysatorpartikel Zeolith enthalten.

3. Verfahren nach einem der Ansprüche 1 bis 2, **dadurch gekennzeichnet, dass** die Regenerierung durch Zufuhr eines Wasserstoff enthaltenden Regenerationsgasstroms durchgeführt wird.

4. Verfahren nach einem der Ansprüche 1 bis 3, **dadurch gekennzeichnet, dass** die inerten Wärmeüberträgerpartikel durch Segregation, Sichten, magnetische Abtrennung, elektrostatische Abtrennung und/oder Sieben abgetrennt werden.

5. Verfahren nach einem der Ansprüche 1 bis 4, **dadurch gekennzeichnet, dass** die Wärmeüberträgerpartikel während oder nach Schritt (b) abgetrennt werden.

6. Verfahren nach einem der Ansprüche 1 bis 5, **dadurch gekennzeichnet, dass** Katalysatorpartikel in der Reaktionszone als Wanderbett, Wirbelbett oder Fließbett vorliegen.

7. Verfahren nach einem der Ansprüche 1 bis 6, **dadurch gekennzeichnet, dass** sich an die Reaktionszone direkt die Regenerationszone zur Regenerierung der Katalysatorpartikel anschließt.

8. Verfahren nach Anspruch 7, **dadurch gekennzeichnet, dass** die Reaktionszone und die Regenerationszone als gemeinsames, zoniertes Wirbelbett betrieben werden.

9. Verfahren nach Anspruch 7 oder 8, **dadurch gekennzeichnet, dass** die Regenerationszone unterhalb der Reaktionszone angeordnet ist und höchstens den gleichen Querschnitt senkrecht zur Hauptströmungsrichtung der Partikel aufweist wie die Reaktionszone.

10. Verfahren nach einem der Ansprüche 7 bis 9, **dadurch gekennzeichnet, dass** sich an die Regenerationszone eine Strippzone anschließt.

11. Verfahren nach Anspruch 10, **dadurch gekennzeichnet, dass** das Strippen in der Strippzone durch Zufuhr eines Wasserstoff enthaltenden Strippgasstroms durchgeführt wird.

12. Verfahren nach Anspruch 10 oder 11, **dadurch gekennzeichnet, dass** die Strippzone unterhalb der Regenerationszone angeordnet ist und höchstens den gleichen Querschnitt senkrecht zur Hauptströmungsrichtung der Partikel aufweist wie die Regenerationszone.

13. Verfahren nach einem der Ansprüche 7 bis 12, **dadurch gekennzeichnet, dass** die Wärmeüberträgerpartikel in der Regenerationszone von den Katalysatorpartikeln abgetrennt werden.

14. Verfahren nach einem der Ansprüche 10 bis 12, **dadurch gekennzeichnet, dass** die Wärmeüberträgerpartikel in der Strippzone von den Katalysatorpartikeln abgetrennt werden.

15. Verfahren nach Anspruch 13 oder 14, **dadurch gekennzeichnet, dass** die inerten Wärmeüberträgerpartikel und die Katalysatorpartikel unterschiedliche Fluidisierungseigenschaften aufweisen und von einander abgetrennt werden, indem durch entsprechendes Einstellen des Regenerationsgasstromes in der Regenerationszone bzw. des Strippgasstromes in der Strippzone die Katalysatorpartikel und die inerten Wärmeüberträgerpartikel unterschiedlich stark fluidisiert werden und entmischen.

16. Verfahren nach einem der Ansprüche 1 bis 15, **dadurch gekennzeichnet, dass** die abgetrennten inerten Wärmeüberträgerpartikel in Schritt (c3) durch Kontakt mit heißem Inertgas, Kontakt mit heißem Verbrennungsabgas, direkte Verbrennung mindestens eines Brennstoffs, Abbrennen von Ablagerungen auf den Wärmeüberträgerpartikeln, Kontakt mit heißen Oberflächen, Einwirken von elektromagnetischen Wellen, elektrisch und/oder durch Induktion erhitzt werden.

17. Verfahren nach einem der Ansprüche 1 bis 15, **dadurch gekennzeichnet, dass** die abgetrennten inerten Wärmeüberträgerpartikel in Schritt (c3) durch Kontakt mit heißem Verbrennungsabgas, direkte Verbrennung mindestens eines Brennstoffs und/oder Abbrennen von Ablagerungen auf den Wärmeüberträgerpartikeln erhitzt werden.

18. Verfahren nach einem der Ansprüche 1 bis 17, **dadurch gekennzeichnet, dass** die abgetrennten Wärmeüberträgerpartikel höchstens 0,1 Gew.-% Katalysatorpartikel, bezogen auf die Gesamtmenge der abgetrennten Partikel, enthalten.

19. Verfahren nach einem der Ansprüche 1 bis 18, **dadurch gekennzeichnet, dass** das Gewichtsverhältnis von Wärmeüberträgerpartikel und Katalysatorpartikel in der Reaktionszone 2 : 1 bis 1 : 10 beträgt.

20. Verfahren nach einem der Ansprüche 1 bis 19, **dadurch gekennzeichnet, dass** die inerten Wärmeüberträgerpartikel ausgewählt sind aus der Gruppe bestehend aus Glaskugeln, Keramikkugeln, Siliziumcarbidpartikel, Al₂O₃-Partikel, Stearitpartikel und Sand.

## Claims

1. A process for carrying out endothermic, heterogeneously catalyzed reactions, which comprises the steps
(a) carrying out the reaction in a reaction zone in the presence of a mixture comprising catalyst particles and inert heat transfer particles,
(b) regeneration of the catalyst particles, comprising
(b1) transfer of the mixture comprising catalyst particles and optionally inert heat transfer particles into a regeneration zone,
(b2) regeneration of the catalyst particles and optionally the inert heat transfer particles in a nonoxidative atmosphere and
(b3) recirculation of the regenerated catalyst particles to the reaction zone and
(c) introduction of heat into the reaction zone, which comprises the steps
(c1) separation of the inert heat transfer particles from the catalyst particles between step (a) and (b), during step (b) or after step (b),
(c2)transfer of the inert heat transfer particles which have been separated off into a heating zone and
(c3)heating of the inert heat transfer particles and recirculation of the heated inert heat transfer particles to the reaction zone,
wherein the reaction in step (a) is the non-oxidative dehydroaromatization of C₁-C₄-aliphatics.

2. The process according to claim 1, wherein the catalyst particles comprise zeolite.

3. The process according to claim 1 or 2, wherein the regeneration is carried out by introduction of a hydrogen-comprising regeneration gas stream.

4. The process according to any of claims 1 to 3, wherein the inert heat transfer particles are separated off by segregation, classification, magnetic separation, electrostatic separation and/or sieving.

5. The process according to any of claims 1 to 4, wherein the heat transfer particles are separated off during or after step (b).

6. The process according to any of claims 1 to 5, wherein catalyst particles are present in the reaction zone as a moving bed or fluidized bed.

7. The process according to any of claims 1 to 6, wherein the regeneration zone for regenerating the catalyst particles directly adjoins the reaction zone.

8. The process according to claim 7, wherein the reaction zone and the regeneration zone are operated as a combined fluidized bed divided into zones.

9. The process according to claim 7 or 8, wherein the regeneration zone is arranged below the reaction zone and has at most the same cross section perpendicular to the main flow direction of the particles as the reaction zone.

10. The process according to any of claims 7 to 9, wherein a stripping zone adjoins the regeneration zone.

11. The process according to claim 10, wherein the stripping in the stripping zone is carried out by introduction of a hydrogen-comprising stripping gas stream.

12. The process according to claim 10 or 11, wherein the stripping zone is arranged below the regeneration zone and has at most the same cross section perpendicular to the main flow direction of the particles as the regeneration zone.

13. The process according to any of claims 7 to 12, wherein the heat transfer particles are separated off from the catalyst particles in the regeneration zone.

14. The process according to any of claims 10 to 12, wherein the heat transfer particles are separated off from the catalyst particles in the stripping zone.

15. The process according to claim 13 or 14, wherein the inert heat transfer particles and the catalyst particles have different fluidization properties and are separated from one another by the catalyst particles and the inert heat transfer particles being fluidized to differing extents and demixing as a result of appropriate setting of the regeneration gas flow in the regeneration zone or of the stripping gas flow in the stripping zone.

16. The process according to any of claims 1 to 15, wherein the inert heat transfer particles which have been separated off are heated in step (c3) by contact with hot inert gas, contact with hot combustion offgas, direct combustion of at least one fuel, burning-off of deposits on the heat transfer particles, contact with hot surfaces, action of electromagnetic waves, electrically and/or by induction.

17. The process according to any of claims 1 to 15, wherein the inert heat transfer particles which have been separated off are heated in step (c3) by contact with hot combustion offgas, direct combustion of at least one fuel and/or burning-off of deposits on the heat transfer particles.

18. The process according to any of claims 1 to 17, wherein the heat transfer particles which have been separated off comprise not more than 0.1% by weight of catalyst particles, based on the total amount of the particles which have been separated off.

19. The process according to any of claims 1 to 18, wherein the weight ratio of heat transfer particles to catalyst particles in the reaction zone is from 2:1 to 1:10.

20. The process according to any of claims 1 to 19, wherein the inert heat transfer particles are selected from the group consisting of glass spheres, ceramic spheres, silicon carbide particles, Al₂O₃ particles, steatite particles and sand.

## Revendications

1. Procédé pour la mise en oeuvre de réactions endothermiques à catalyse hétérogène, comprenant les étapes
(a) exécution de la réaction dans une zone de réaction en présence d'un mélange contenant des particules de catalyseur et des particules caloporteuses inertes,
(b) régénération des particules de catalyseur comprenant
(b1) transfert dans une zone de régénération du mélange contenant des particules de catalyseur et éventuellement des particules caloporteuses inertes,
(b2) régénération en atmosphère non oxydante des particules de catalyseur et éventuellement des particules caloporteuses inertes et
(b3) renvoi dans la zone de réaction des particules de catalyseur régénérées, et
(c) introduction de chaleur dans la zone de réaction, comprenant les étapes
(c1) séparation des particules caloporteuses inertes d'avec les particules de catalyseur entre l'étape (a) et l'étape (b), pendant l'étape (b) ou après l'étape (b),
(c2) transfert dans une zone de chauffage des particules caloporteuses inertes séparées et
(c3) chauffage des particules caloporteuses inertes et renvoi dans la zone de réaction des particules caloporteuses inertes chauffées,
**caractérisé en ce que** pour ce qui est de la réaction dans l'étape (a) il s'agit de la déshydroaromatisation non oxydante de composés aliphatiques en C₁-C₄.

2. Procédé selon la revendication 1, **caractérisé en ce que** les particules de catalyseur comprennent une zéolithe.

3. Procédé selon la revendication 1 ou 2, **caractérisé en ce que** la régénération est effectuée par introduction d'un courant de gaz de régénération contenant de l'hydrogène.

4. Procédé selon l'une quelconque des revendications 1 à 3, **caractérisé en ce que** les particules caloporteuses inertes sont séparées par ségrégation, criblage, séparation magnétique, séparation électrostatique et/ou tamisage.

5. Procédé selon l'une quelconque des revendications 1 à 4, **caractérisé en ce qu'**on sépare les particules caloporteuses pendant ou après l'étape (b).

6. Procédé selon l'une quelconque des revendications 1 à 5, **caractérisé en ce que** dans la zone de réaction les particules de catalyseur se trouvent sous forme de lit mobile, lit tourbillonnaire ou lit fluidisé.

7. Procédé selon l'une quelconque des revendications 1 à 6, **caractérisé en ce qu'**à la zone de réaction fait directement suite la zone de régénération pour la régénération des particules de catalyseur.

8. Procédé selon la revendication 7, **caractérisé en ce qu'**on fait fonctionner la zone de réaction et la zone de régénération sous forme de lit tourbillonnaire commun divisé en zones.

9. Procédé selon la revendication 7 ou 8, **caractérisé en ce que** la zone de régénération est disposée au-dessous de la zone de réaction et présente au maximum la même section transversale, perpendiculairement au sens de circulation principal des particules, que la zone de réaction.

10. Procédé selon l'une quelconque des revendications 7 à 9, **caractérisé en ce qu'**à la zone de régénération fait suite une zone de strippage.

11. Procédé selon la revendication 10, **caractérisé en ce que** le strippage dans la zone de strippage est effectué par introduction d'un courant de gaz d'entraînement contenant de l'hydrogène.

12. Procédé selon la revendication 10 ou 11, **caractérisé en ce que** la zone de strippage est disposée au-dessous de la zone de régénération et présente au maximum la même section transversale, perpendiculairement au sens de circulation principal des particules, que la zone de régénération.

13. Procédé selon l'une quelconque des revendications 7 à 12, **caractérisé en ce que** dans la zone de régénération les particules caloporteuses sont séparées d'avec les particules de catalyseur.

14. Procédé selon l'une quelconque des revendications 10 à 12, **caractérisé en ce que** dans la zone de strippage les particules caloporteuses sont séparées d'avec les particules de catalyseur.

15. Procédé selon la revendication 13 ou 14, **caractérisé en ce que** les particules caloporteuses inertes et les particules de catalyseur présentent des propriétés de fluidisation différentes et sont séparées les unes des autres par le fait que les particules de catalyseur et les particules caloporteuses inertes sont fluidisées et dissociées à des degrés différents par réglage convenable du courant de gaz de régénération dans la zone de régénération ou respectivement du courant de gaz d'entraînement dans la zone de strippage.

16. Procédé selon l'une quelconque des revendications 1 à 15, **caractérisé en ce que** les particules caloporteuses inertes séparées sont chauffées dans l'étape (c3) par contact avec un gaz inerte chaud, contact avec un gaz chaud de combustion, combustion directe d'au moins un combustible, brûlage de dépôts sur les particules caloporteuses, contact avec des surfaces chaudes, action d'ondes électromagnétiques, électriquement et/ou par induction.

17. Procédé selon l'une quelconque des revendications 1 à 15, **caractérisé en ce que** les particules caloporteuses inertes séparées sont chauffées dans l'étape (c3) par contact avec un gaz chaud de combustion, combustion directe d'au moins un combustible et/ou brûlage de dépôts sur les particules caloporteuses.

18. Procédé selon l'une quelconque des revendications 1 à 17, **caractérisé en ce que** les particules caloporteuses séparées contiennent au maximum 0,1 % en poids de particules de catalyseur, par rapport à la quantité totale des particules séparées.

19. Procédé selon l'une quelconque des revendications 1 à 18, **caractérisé en ce que** dans la zone de réaction le rapport pondéral des particules caloporteuses et des particules de catalyseur vaut de 2 : 1 à 1 : 10.

20. Procédé selon l'une quelconque des revendications 1 à 19, **caractérisé en ce que** les particules caloporteuses inertes sont choisies dans le groupe constitué par des billes de verre, des billes de céramique, des particules de carbure de silicium, des particules d'Al₂O₃ et le sable.
